# EUROPEAN PATENT SPECIFICATION

(11) **EP 1 117 424 B1**
(45) Date of publication and mention of the grant of the patent: **31.08.2005**
(21) Application number: 99950037.4
(22) Date of filing: 29.09.1999
(51) Int. Cl.: A61K 38/30, A61P 35/00

(54) **NULL IGF FOR THE TREATMENT OF CANCER**
NULL IGF ZUR KREBSBEHANDLUNG
IGF INACTIF AUX FINS DU TRAITEMENT DU CANCER

(30) Priority: 02.10.1998 US 102747 P; 20.09.1999 US 399120
(43) Date of publication of application: 25.07.2001
(73) Proprietor: CELTRIX PHARMACEUTICALS, INC., Glen Allen, VA 23060 (US)
(72) Inventor: MASCARENHAS, Desmond, Los Altos, CA 94022 (US)
(74) Representative: Wright, Simon Mark
(86) International application number: PCT/US1999/022681
(87) International publication number: WO 2000/020023

(56) References cited:
- WO-A-95/16703
- WO-A-98/36764
- US-A- 5 310 742
- PASCASIO F M ET AL: "METHIMAZOLE IN THE TREATMENT OF HEPATO CELLULAR CARCINOMA A PILOT STUDY" PHILIPPINE JOURNAL OF INTERNAL MEDICINE 1981, vol. 19, no. 2, 1981, pages 80-84, XP002135043 ISSN: 0556-0071

## Description

### TECHNICAL FIELD

The invention relates to the field of treatment of cancer, and particularly to the use of null IGF for the treatment of cancer.

### BACKGROUND ART

Growth factors are polypeptides which stimulate a wide variety of biological responses (e.g. DNA synthesis, cell division, expression of specific genes, etc.) in a defined population of target cells. A variety of growth factors have been identified, including the transforming growth factor beta family (TGF-βs), epidermal growth factor and transforming growth factor alpha (the TGF-αs), the platelet-derived growth factors (PDGFs), the fibroblast growth factor family (FGFs) and the insulin-like growth factor family (IGFs), which includes IGF-I and IGF-II. Many growth factors have been implicated in the pathogenesis of cancer.

IGF-I and IGF-II (the "IGFs")are related in amino acid sequence and structure, with each polypeptide having a molecular weight of approximately 7.5 kilodaltons (kDa). IGF-I mediates the major effects of growth hormone, and is thus the primary mediator of growth after birth. IGF-I has also been implicated in the actions of various other growth factors, since the treatment of cells with such growth factors leads to increased production of IGF-I. In contrast, IGF-II is believed to have a major role in fetal growth. Both IGF-I and IGF-II have insulin-like activities (hence their names), and are mitogenic (stimulate cell division).

IGF-I has been found to stimulate the growth of cells from a number of different types of cancer (Butler et al., 1998 *Cancer Res.* **58**(14):3021-3027; Favoni RE, et al., 1998, *Br. J Cancer* **77**(12): 2138-2147). Additionally, IGF-I has additionally been found to exert anti-apoptotic effects on a number of different cell types, including tumor cells (Giuliano M, et al., 1998 *Invest Ophthalmol. Vis. Sci.* **39**(8): 1300-1311; Zawada WM, et al., 1998, *Brain Res.* **786**(1-2): 96-103; Kelley KW, et al., 1998, *Ann. N. Y. Acad. Sci.* **840**: 518-524; Toms SA, et al., 1998, *J. Neurosurg.* **88**(5): 884-889; Xu F, et al., 1997, *Br. J Haematol.* **97**(2): 429-440). U.S. Patent No. 5,681,818 claims the administration of IGFBP-3 for the treatment of cancer.

A number of variant forms of IGF-I have been created which have altered binding characteristics for the IGF receptors, the insulin receptor, or IGFBP's (Cascieri *et al*. (1988) *Biochemistry* 27:3229-3233 and (1989) *J. Biol. Chem.* **264**:2199-2202; Bayne *et al*. (1990) *J. Biol. Chem.* **265**:15648-15652); Baxter *et al*. (1992) *J. Biol. Chem.* **267**:60-65). Additionally, International Patent Application No. WO 97/39032 discloses the use of certain variant forms of IGF-I for the treatment of conditions where increased IGF-I activity is desired, such as diabetes, osteoporosis, and the like. The variant forms of IGF-I are proposed to displace IGF-I from IGFBP, resulting in increased IGF-I activity.

Almost all IGF circulates in a non-covalently associated complex of IGF-I, insulin-like growth factor binding protein 3 (IGFBP-3) and a larger protein subunit termed the acid labile subunit (ALS), such that very little free IGF-I is detectable. The ternary complex is composed of equimolar amounts of each of the three components. ALS has no direct IGF-binding activity and appears to bind only to the IGF/IGFBP-3 complex (Baxter et al., *J. Biol. Chem.* **264**(20):11843-11848, 1989), although some reports suggest that IGFBP-3 can bind to rat ALS in the absence of IGF (Lee et al., *Endocrinology* **136**:4982-4989, 1995). The ternary complex of IGF/IGFBP-3/ALS has a molecular weight of approximately 150 kDa and has a substantially increased half-life in circulation when compared to binary IGF/IGFBP-3 complex or IGF alone (Adams et al., *Prog. Growth Factor Res*. **6**(2-4):347-356; presented October 1995, published 1996). This ternary complex is thought to act "as a reservoir and a buffer for IGF-I and IGF-II preventing rapid changes in the concentration of free IGF" (Blum *et al*. (1991), "Plasma IGFBP-3 Levels as Clinical Indicators" in MODERN CONCEPTS OF INSULIN-LIKE GROWTH FACTORS, pp. 381-393, E.M. Spencer, ed., Elsevier, New York). While there is essentially no excess (unbound) IGFBP-3 in circulation, a substantial excess of free ALS does exist (Baxter, *J. Clin. Endocrinol. Metab.* **67**:265-272, 1988).

It should be noted that, while IGFBP-3 is the most abundant of the IGF binding proteins ("IGFBPs"), at least five other distinct IGFBPs have been identified in various tissues and body fluids. Although these proteins bind IGFs, they originate from separate genes and have distinct amino acid sequences. Unlike IGFBP-3, other circulating IGFBPs are not saturated with IGFs. IGFBP-3 and IGFBP-5 are the only known IGFBPs which can form the 150 kDa ternary complex with IGF and ALS. The IGF and ALS binding domains of IGFBP-3 are thought to be in the N-terminal portion of the protein, as N-terminal fragments of the protein isolated from serum retain these binding activities. However, some of the other IGFBPs have also been suggested for use in combination with IGF-I as therapeutics.

In addition to its role as the major carrier protein for IGF in serum, IGFBP-3 has been recently shown to have a number of different activities. IGFBP-3 can bind to an as-yet unidentified molecule on the cell surface, where it can inhibit the activity of exogenously-added IGF-I (Karas et al., 1997, *J. Biol. Chem.* **272**(26):16514-16520). Although the binding of IGFBP-3 to cell surfaces can be inhibited by heparin, the unidentified cell surface binding molecule is unlikely to be a heparin-like cell surface glycosaminoglycan, because enzymatic removal of heparin glycosaminoglycans has no effect on IGFBP-3 cell surface binding (Yang et al., 1996, *Endocrinology* **137**(10):4363-4371). It is not clear if the cell surface binding molecule is the same or different than the IGFBP-3 receptor that was identified by Leal et al. (1997, *J*. *Biol. Chem*. **272**(33):20572-20576), which is identical to the type V transforming growth factor-beta (TGF-β) receptor.

IGFBP-3 has also been found to promote apoptosis. Interestingly, IGFBP-3 has been shown to promote apoptosis in cells with and without functional type 1 IGF receptors (Nickerson et al., 1997, *Biochem. Biophys. Res. Comm.* **237**(3):690-693; Rajah et al., 1997, *J. Biol. Chem.* **272**(18):12181-12188). However, there are conflicting reports as to whether apoptosis is induced by full length IGFBP-3 or a proteolytic fragment of IGFBP-3 (Rajah et al., *ibid;* Zadeh et al., 1997, *Endocrinology* **138**(7):3069-3072).

IGF-I and IGFBP-3 may be purified from natural sources or produced by recombinant means. For instance, purification of IGF-I from human serum is well known in the art (Rinderknecht *et al*. (1976) *Proc. Natl. Acad. Sci. USA* **73**:2365-2369). Production of IGF-I by recombinant processes is shown in EP 0 128 733, published in December of 1984. IGFBP-3 may be purified from natural sources using a process such as that shown in Baxter *et al*. (1986, *Biochem. Biophys. Res. Comm.* **139**:1256-1261). Alternatively, IGFBP-3 may be synthesized by recombinantly as discussed in Sommer *et al*., pp. 715-728, MODERN CONCEPTS OF INSULIN-LIKE GROWTH FACTORS (E.M. Spencer, ed., Elsevier, New York, 1991). Recombinant IGFBP-3 binds IGF-I in a 1:1 molar ratio.

Topical administration of IGF-I/IGFBP-3 complex to rat and pig wounds is significantly more effective than administration of IGF-I alone (*Id*.). Subcutaneous administration of IGF-I/IGFBP-3 complex to hypophysectomized, ovariectomized, and normal rats, as well as intravenous administration to cynomolgus monkeys, "substantially prevents the hypoglycemic effects" of IGF-I administered alone (*Id*.).

The use of IGF/IGFBP-3 complex has been suggested for the treatment of a wide variety of disorders (see, for example, U.S. Patents Nos. 5,187,151, 5,527,776, 5,407,913, 5,643,867, 5,681,818 and 5,723,441, as well as International Patent Applications Nos. WO 95/03817, WO 95/13823, and WO 96/02565. IGF-I/IGFBP-3 complex is also under development by Celtrix Pharmaceuticals, Inc., as a treatment for several indications, including recovery from burns and recovery from hip fracture surgery.

While there are a large number of cytotoxic drugs available for the treatment cancer, these drugs are generally associated with a variety of serious side effects, including alopecia, leukopenia, mucositis. Accordingly, there is a need in the art for cancer therapies that do not induce the serious side effects associated with conventional cytotoxic chemotherapy.

WO-A-98/36764 is concerned with treating psychological and metabolic disorders. WO-A-95/16703 is concerned with IGF-1 analogs.

### DISCLOSURE OF THE INVENTION

The inventor has surprisingly found that null IGF-I, administered in the absence of IGFBP-3, is effective in alleviating the symptoms of cancer, whereas null IGF-I administered as a complex with IGFBP-3 is ineffective. This finding was unexpected because IGFBP-3 is known to substantially increase the half-life of IGF-I and to increase the efficacy of IGF-I (Sommer *et al., supra*), so it was expected that null IGF-I/IGFBP-3 complex would be more efficacious in alleviating the symptoms of cancer than null IGF-I administered in the absence of IGFBP-3.

The object of the invention is defined in the claims.

Disclosed herein is the use of uncomplexed null insulin-like growth factor I (IGF-I) in the manufacture of a medicament for alleviating the symptoms of cancer and/or slowing progression of cancer. An effective amount of null IGF is to be administered to a subject having cancer, thereby alleviating the symptoms of the cancer.

In another embodiment, a thyroid axis antagonist is to be administered with the null IGF-I to the subject having cancer.

### Definitions

As used herein, the term "null IGF-I" refers to IGF-I which has amino acid sequence alterations at one or more sites in the molecule. Null IGF-I retains its ability to bind IGFBP-3, but is altered in its receptor binding and/or activating properties (e.g., having little or no binding to the type 1 IGF receptor while maintaining its binding activities for the type 2 IGF receptor and the insulin receptor). A preferred null IGF-I has substantially reduced binding to both types of the IGF receptor and the insulin receptor. Descriptions of null IGF-I's may be found in Cascieri *et al*. (1988) *Biochemistry* 27:3229-3233; (1989) *J. Biol. Chem.* **264**:2199-2202), Bayne *et al*. (1990) *J. Biol. Chem.* **265**:15648-15652) and Baxter *et al*. (1992) *J. Biol. Chem.* **267**:60-65). Examples of null IGF-I include variants in which one or more of IGF-I's tyrosine residues (*i*.*e*., residues 24, 31, or 60) are replaced with non-aromatic residues (*i*.*e*., other than tyrosine, phenylalanine or tryptophan), variants where amino acid residues 49, 50, 51, 53, 55 and 56 are altered (for example, where residues 49-50 are altered to Thr-Ser-Ile or where residues 55-56 are altered to Tyr-Gln), and combinations thereof.

The term "thyroid axis antagonist" refers to a compound which acts to decrease thyroid hormone activity in a subject. Thyroid axis antagonists include 6-n-propyl-2-thiouracil (propylthiouracil or PTU), methimazole, carbimazole, and other compounds known to the art to reduce thyrotropic hormones, thyroid hormones, or thyroid receptor signaling.

### BRIEF DESCRIPTION OF THE DRAWINGS

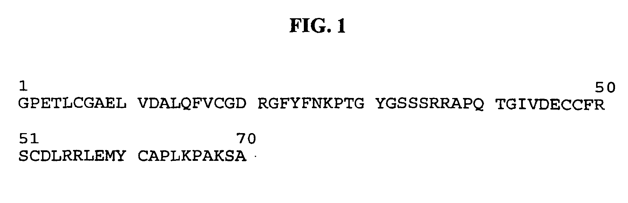

FIG. 1 shows the amino acid sequence of native human IGF-I in single-letter amino acid code.
FIG. 2 depicts the results of the experiment described in Example 2. The percentage of surviving animals (y-axis) is plotted against time after xenograft implantation (x-axis). The data for null₆₀ IGF-I, null60 complex and control treated groups are represented by open circles, open diamonds and open squares, respectively.

### BEST MODE FOR CARRYING OUT THE INVENTION

Disclosed herein are new methods for the treatment of cancer. An effective amount of null IGF-I is to be administered to a subject suffering from cancer, thereby alleviating the symptoms of the cancer. Null IGF-I slows the growth rate of cancer, thereby alleviating the symptoms of, or slowing the progression of the cancer. While not wishing to be bound by any particular theory, the inventor believes that the administration of null IGF-I displaces native IGF-I from complexes with binding proteins, particularly binding proteins other than IGFBP-3 (e.g., IGFBP-2), resulting in reduced IGF-I activity, which reduces growth of the tumor and renders the tumor cells more responsive to apoptotic signals.

The inventors have surprisingly found that administration of null IGF-I is substantially more effective at slowing tumor growth than the administration of null IGF-I as a complex with IGFBP-3. This was surprising because it is well known that uncomplexed IGF-I has a significantly shorter half-life than IGF-I administered as a complex with IGFBP-3 and that IGF-I administered as a complex with IGFBP-3 is more effective than uncomplexed IGF-I.

Null IGF-I may be used to treat any cancer, preferably carcinomas such as breast, prostate, cancer and lung cancers.

Null IGF-I for use in accordance with the instant invention may be derived from any species, although species-matched null IGF-I (*i*.*e*., null IGF-I based on the native sequence from the same species as the subject to which the IGF-I is to be administered) is preferred. Null IGF-I for use in the instant invention is uncomplexed null IGF-I, that is, administered in the absence of IGFBP-3 (*i*.*e*., is not administered as null IGF-I/IGFBP-3 complex), and is preferably administered in the absence of any IGF binding protein.

The null IGF-I is normally produced by recombinant methods, which allow the production of all possible variants in IGF-I sequence. Techniques for the manipulation of recombinant DNA are well known in the art, as are techniques for recombinant production of proteins (see, for example, in Sambrook et al., MOLECULAR CLONING: A LABORATORY MANUAL, Vols. 1-3 (Cold Spring Harbor Laboratory Press, 2 ed., (1989); or F. Ausubel et al., CURRENT PROTOCOLS IN MOLECULAR BIOLOGY (Green Publishing and Wiley-Interscience: New York, 1987) and periodic updates).

Preferably, the null IGF-I is produced using a bacterial cell strain as the recombinant host cell. An expression construct (*i*.*e*., a DNA sequence comprising a sequence encoding the desired null IGF-I operably linked to the necessary DNA sequences for proper expression in the host cell, such as a promoter and/or enhancer elements at the 5' end of the construct and terminator elements in the 3' end of the construct) is introduced into the host cell. The DNA sequence encoding the null IGF may optionally linked to a sequence coding another protein (a "fusion partner"), to form a fusion protein. Preferably, the DNA sequence encoding the null IGF-I is linked to a sequence encoding a fusion partner as described in International Patent Application No. WO 94/04076. The expression construct may be an extrachromosomal construct, such as a plasmid or cosmid, or it may be integrated into the chromosome of the host cell, for example as described in International Patent Application No. WO 96/40722.

Null IGF-I is preferably administered by parenteral administration, including but not limited to intravenous (IV), intraperitoneal (IP), intramuscular (IM), subcutaneous (SC), intradermal (ID), transdermal, inhaled, and intranasal routes. IV, IP, IM, and ID administration may be by bolus or infusion administration. For SC administration, administration may be by bolus, infusion, or by implantable device, such as an implantable minipump (*e.g.*, osmotic or mechanical minipump) or slow release implant. The null IGF-I may also be delivered in a slow release formulation adapted for IV, IP, IM, ID or SC administration. Inhaled null IGF-I is preferably delivered in discrete doses (e.g., via a metered dose inhaler adapted for protein delivery). Administration of null IGF-I via the transdermal route may be continuous or pulsatile.

For parenteral administration, compositions of null IGF-I may be in dry powder, semi-solid or liquid formulations. For parenteral administration by routes other than inhalation, the null IGF-I is preferably administered in a liquid formulation. Null IGF-I formulations may contain additional components such as salts, buffers, bulking agents, osmolytes, antioxidants, detergents, surfactants, and other pharmaceutical excipients as are known in the art.

Null IGF-I is administered to subjects having cancer at a dose of about 0.01 to about 50 mg/kg/day, more preferably about 0.1 to about 20 mg/kg/day, more preferably 0.5 to about 15 mg/kg/day, even more preferably about 1 to about 10 mg/kg/day.

In certain embodiments, the null IGF-I is to be administered to the subject with a thyroid axis antagonist. The administration of the two compounds may be simultaneous, overlapping, or separated in time, as long as the subject experiences exposure to both compounds at the same time. Where the two compounds are formulated for the same route and schedule of administration, the administration is preferably simultaneous or nearly simultaneous (*e.g.*, concurrent or serial injections). However, in some embodiments, the routes and schedules of administration for the two compounds will be different, making simultaneous administration inconvenient. A subject will be considered to have been administered null IGF-I and a thyroid axis antagonist if the subject experiences simultaneous systemic exposure to both compounds, regardless of when or how the compounds were administered.

When the administration of thyroid axis antagonists is required with the null IGF-I, the dose of the thyroid axis antagonist is normally titrated for the individual subject, as is well known in the art. Induction of frank hypothyroidism is not required, though in some cases it may be found advantageous, for the proper working of this invention. Thyroid axis antagonists are generally administered at an intermediate dose, and the patient observed for the onset of hypothyroidism. Hypothyroidism may be recognized by its well known symptoms, including (in adults) lethargy, constipation, cold intolerance, menorrhagia (in women of reproductive age), reduced intellectual and motor activity, dry hair, dry skin, muscle aches, reduced auditory acuity, and deepening and hoarsening of the voice. In extreme cases, florid myxedema may be present, as indicated by a dull, expressionless face, sparse hair, periorbital puffiness, enlarged tongue and pale, cool skin which feels rough and doughy. The thyroid antagonist dose should be reduced if florid myxedema appears, to avoid the possibility of myxedema coma, a serious and frequently fatal condition. Upon the appearance of the signs of hypothyroidism which fall short of florid myxedema, the dose of the thyroid axis antagonist may be reduced to the point at which the symptoms of hypothyroidism resolve, as will be understood by one of skill in the art.

Thyroid axis antagonists may be produced in any formulation known to the art, including parenteral and oral dosage forms. Oral formulations are preferred, but parenteral formulations are also acceptable, and may be more convenient in an in-patient setting. Formulations for parenteral administration are generally formulated as liquids, but may also be in gel or solid depot form. Formulations for oral administration are generally in tablet or capsule form, although syrups and liquids are also acceptable. Formulations of thyroid axis antagonists generally include excipients, such as salts, buffers, bulking agents, detergents, binding agents, surfactants, stabilizers, preservatives, anti-oxidants, lubricants, coating agents, and other pharmaceutically acceptable excipients as are known in the art.

The dosage of thyroid axis antagonist should be adjusted according to the identity, formulation and route of administration of the thyroid axis antagonist which is administered with the null IGF-I, as is known in the art. Where the thyroid axis antagonist is propylthiouracil, the dose of propylthiouracil may be from 1 to 400 mg/day. A subject is normally initiated with a dose of 50 to 400 mg/day, typically divided into three equal doses, and maintained at 50 to 100 mg/day divided into two or three equal doses. For methimazole and carbimazole, the dose may be from 0.1 to 50 mg/day. Typically, a subject is initiated with 5 to 50 mg/day, and maintained on 1 to 5 mg/day.

### EXAMPLES

### Example 1: Pharmacokinetics of null IGF-I

The pharmacokinetics of three test articles were assayed: (1) a recombinant human null IGF-I (Y60L IGF-I), in which the normal tyrosine residue at position 60 had been substituted with leucine; variant recombinant human IGFBP-3 (N109D,N172D IGFBP-3), in which the asparagine residues normally at positions 109 and 172 were substituted with aspartate; and Y60L IGF-I/N109D,N172D IGFBP-3 complex at a 1:1 molar ratio. Nine mice, each weighing approximately 20-25 grams were utilized in the study. Mouse were housed in standard mouse cages, one per cage, and fed with water and mouse chow *ad libitum.*

Y60L IGF-1, N109D,N172D IGFBP-3, and Y60L IGF-I/N109D,N172D IGFBP-3 complex were dissolved in 50 mM sodium acetate, pH 5.5, 108 mM NaCl, at 2, 8, and 10 mg/ml, respectively. The animals were randomly divided into three groups of three animals each.

The animals received 100 µl of the undiluted appropriate test article in a single subcutaneous bolus. Blood samples (approximately 50 µl each) were collected by eye-bleeds at 0.08, 1, 2, 4, and 8 house after the injection. Serum was isolated from each sample, and assayed for human IGF-I and IGFBP-3, as appropriate, using commercially-available immunoassay kits obtained from DSL (Webster, TX) according to the manufacturer's instructions. The approximate area under the curve (AUC) was calculated by multiplying concentrations by the time periods between samples for each animal, and mean AUC's (in arbitrary units) were determined for each group. The results are shown in Table I ("nd" indicates that the assay was not performed).

**TABLE I**

| Test Article | Dose (mg/kg) | AUC_{IGF-I} | AUC_{IGFBP-3} |
|---|---|---|---|
| Y60L IGF-I | 10 | 2,597 | nd |
| N109D/N172D IGFBP-3 | 40 | nd | 15,480 |
| Y60L IGF-I/N109D,N172D IGFBP-3 complex | 50 | 7,505 | 37,797 |

The data clearly indicates that, as expected, administration of null IGF-I as a complex with IGFBP-3 substantially increases systemic exposure to null IGF-I, as indicated by the substantial increase in AUC.

### Example 2: Treatment of Prostate Cancer Tumors with Null IGF-I

Y60L IGF-I and Y60L IGF-I/N109D,N172D IGFBP-3 complex were tested for anti-tumor activity in nude mice. 36 mice which had been implanted with PC-3 (human prostate) tumor xenografts were obtained from the Goodwin Cancer Institute (Plantation, FL). Each mouse had received a subcutaneous xenograft of approximately 4-6 mm³ solid PC-3 tumor.

Three different test articles were employed in this experiment: 2 mg/kg/day Y60L IGF-I ("null₆₀ IGF-I") dissolved in 50 mM sodium acetate, pH 5.5, 108 mM sodium chloride ("vehicle"); 10 mg/kg/day Y60L IGF-I/N109D,N172D IGFBP-3 complex ("null₆₀ IGF-I complex") dissolved in vehicle; and vehicle alone ("control"). The test articles were administered near the xenograft site twice each weekday (Monday through Friday) and once per weekend day (Saturday and Sunday) by subcutaneous bolus injection. Test articles were administered from day 15 after implantation ("Day 15") until the animal was sacrificed.

Animals were sacrificed at day 57 after implantation ("Day 57") or earlier if tumor volume exceeded 2000 mm³. Tumor growth was measured three times per week using calipers. Statistical analysis was performed using a two-tailed t test.

Any animal that did not show evidence of tumor growth by Day 23 was eliminated from the study. 12 null₆₀ IGF-I, 9 null₆₀ IGF-I complex, and 11 control treated mice remained in the study after Day 23.

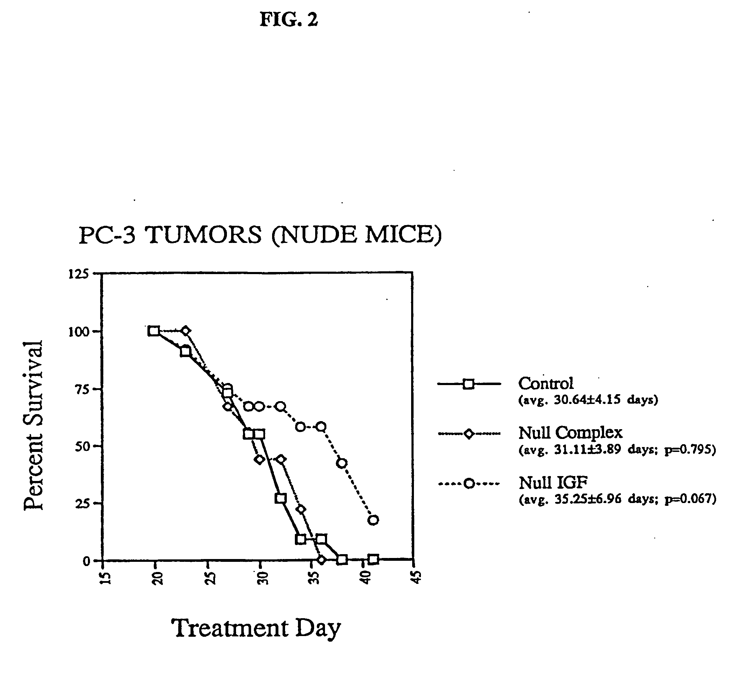

Results of this experiment are depicted graphically in FIG. 2. Survival of the control and null₆₀ complex animals were very similar (average survival 30.6 days and 31.1 days, respectively, p=0.795). However, mice treated with null₆₀ IGF-I had substantially greater survival, with an average survival of 35.25 days at the end of the study (p=0.067 compared with control). It should be noted that the average survival of the null₆₀ IGF-I treated mice was underestimated by this experiment, as two null₆₀ IGF-I treated mice had tumors of less than 2000 mm³ at the end of the study. Inspection of FIG. 2 also reveals that median survival was substantially increased for mice receiving null₆₀ IGF-I.

The present invention has been detailed both by direct description and by example. Equivalents and modifications of the present invention will be apparent to those skilled in the art, and are encompassed within the scope of the invention.

## Claims

1. Use of uncomplexed null insulin-like growth factor I (IGF-I) in the manufacture of a medicament for alleviating the symptoms of cancer and/or slowing progression of a cancer.

2. Use according to claim 1 wherein the null IGF-1 is altered at position 49, 50, 51, 53, 55 and/or 56.

3. Use according to claim 1 or 2, wherein positions 49 to 50 of the null IGF-I are altered to Thr-Ser-Ile and/or positions 55 to 56 to Tyr-Gln.

4. Use according to any one of the preceding claims wherein position 60 of the null IGF-I is altered to a non-aromatic residue.

5. Use according to claim 4 wherein the non-aromatic residue is a leucine residue.

6. Use according to any one of the preceding claims wherein one or more of positions 24 and 31 of the null IGF-I are additionally altered to a non-aromatic residue.

7. Use according to any one of the preceding claims, wherein position 24 of the null IGF-I is altered to a non-aromatic residue.

8. Use according to any one of the preceding claims wherein medicament is to be administered so that the subject receiving it receives about 0.01 to about 50 milligrams per kilogram total body weight per day (mg/kg/day) of the null IGF-I.

9. Use according to any one of the preceding claims wherein the medicament also contains a thyroid axis antagonist.

10. Use according to claim 9 wherein the thyroid axis antagonist is propylthiouracil, methimazole or carbimazole.

11. Use according to any one of the preceding claims, wherein the cancer is breast, prostate, colon and/or lung cancer.

12. A product containing a null IGF-I as defined in any one of claims 1 to 8 and a thyroid axis antagonist as defined in claim 9 and/or 10 for simultaneous, separate or sequential use in alleviating the symptoms of cancer and/or slowing progression of a cancer.

13. A product according to claim 12 wherein the null IGF-1 is to be administered at about 0.01 to about 50 milligrams per kilogram total body weight per day (mg/kg/day).

14. A product according to claim 12 or 13, wherein the cancer is breast, prostate, colon and/or lung cancer.

## Patentansprüche

1. Verwendung eines unkomplexierten Null-Insulin-ähnlichenWachtumsfaktors I (IGF-I) für die Herstellung eines Medikaments zur Linderung von Krebssymptomen und/oder zur Verlangsamung der Krebsentwicklung.

2. Verwendung nach Anspruch 1, wobei der Null-IGF-I an Position 49, 50, 51, 53, 55 und/oder 56 verändert ist.

3. Verwendung nach Anspruch 1 oder 2, wobei die Positionen 49 bis 50 des Null-IGF-I zu Thr-Ser-Ile und/oder die Positionen 55 bis 56 zu Tyr-Gln verändert sind.

4. Verwendung nach einem der vorangehenden Ansprüche, wobei die Position 60 des Null-IGF-I zu einem nicht-aromatischen Rest verändert ist.

5. Verwendung nach Anspruch 4, wobei der nicht-aromatische Rest ein Leucinrest ist.

6. Verwendung nach einem der vorangehenden Ansprüche, wobei eine oder mehrere der Positionen 24 und 31 des Null-IGF-I zusätzlich zu einem nicht-aromatischen Rest verändert sind.

7. Verwendung nach einem der vorangehenden Ansprüche, wobei Position 24 des Null-IGF-I zu einem nicht-aromatischen Rest verändert ist.

8. Verwendung nach einem der vorangehenden Ansprüche, wobei das Medikament so zu verabreichen ist, daß der Patient etwa 0,01 bis 50 Milligramm pro Kilogramm des gesamten Körpergewichts pro Tag (mg/kg/Tag) des Null-IGF-I erhält.

9. Verwendung nach einem der vorangehenden Ansprüche, wobei das Medikament zusätzlich einen Thyroidachse-Antagonisten enthält.

10. Verwendung nach Anspruch 9, wobei der Thyroidachse-Antagonist Propylthiouracil, Methimazol oder Carbimazol ist.

11. Verwendung nach einem der vorangehenden Ansprüche, wobei der Krebs Brustkrebs, Prostatakrebs, Darmkrebs und/oder Lungenkrebs ist.

12. Produkt, welches ein Null-IGF-I, wie in einem der Ansprüche 1 bis 8 definiert und einen Thyroidachse-Antagonisten wie in Anspruch 9 und/oder 10 definiert, enthält, für die gleichzeitige, getrennte oder sequentielle Verwendung zur Linderung von Krebssymptomen und/oder zur Verlangsamung der Krebsentwicklung.

13. Produkt nach Anspruch 12, wobei der Null-IGF-I mit etwa 0,01 bis etwa 50 Milligramm pro Kilogramm des Gesamtkörpergewichts pro Tag (mg/kg/Tag) zu verabreichen ist.

14. Produkt nach Anspruch 12 oder 13, wobei der Krebs Brustkrebs, Prostatakrebs, Darmkrebs und/oder Lungenkrebs ist.

## Revendications

1. Utilisation de facteur de croissance analogue à l'insuline I (IGF-I) nul non complexé dans la fabrication d'un médicament pour atténuer les symptômes du cancer et/ou ralentir l'évolution d'un cancer.

2. Utilisation selon la revendication précédente, dans laquelle l'IGF-1 nul est altéré en position 49, 50, 51, 53, 55 et/ou 56.

3. Utilisation selon la revendication 1 ou 2, dans laquelle les positions 49 à 50 de l'IGF-I nul sont altérées en Thr-Ser-Ile et/ou les positions 55 à 56 en Tyr-Gln.

4. Utilisation selon l'une quelconque des revendications précédentes, dans laquelle la position 60 de l'IGF-I nul est altérée en un résidu non aromatique.

5. Utilisation selon la revendication 4, dans laquelle le résidu non aromatique est un résidu leucine.

6. Utilisation selon l'une quelconque des revendications précédentes, dans laquelle une ou plusieurs des positions 24 et 31 de l'IGF-I nul sont en plus altérées en un résidu non aromatique.

7. Utilisation selon l'une quelconque des revendications précédentes, dans laquelle la position 24 de l'IGF-I nul est altérée en un résidu non aromatique.

8. Utilisation selon l'une quelconque des revendications précédentes, dans laquelle le médicament doit être administré de telle sorte que le sujet le recevant reçoive d'environ 0,01 à environ 50 milligrammes par kilogramme de poids corporel par jour (mg/kg/jour) de l'IGF-I nul.

9. Utilisation selon l'une quelconque des revendications précédentes, dans laquelle le médicament contient également un antagoniste d'axe thyroïdien.

10. Utilisation selon la revendication 9, dans laquelle l'antagoniste d'axe thyroïdien est le propylthio-uracile, le méthimazole ou le carbimazole.

11. Utilisation selon l'une quelconque des revendications précédentes, dans laquelle le cancer est un cancer du sein, de la prostate, du colon et/ou du poumon.

12. Produit contenant un IGF-I nul tel qu'il est défini dans l'une quelconque des revendications 1 à 8, et un antagoniste d'axe thyroïdien tel que défini dans la revendication 9 et/ou 10 pour une utilisation simultanée, séparée ou séquentielle dans l'atténuation des symptômes d'un cancer et/ou le ralentissement de l'évolution d'un cancer.

13. Produit selon la revendication 12, dans lequel l'IGF-nul doit être administré à environ 0,01 à environ 50 milligrammes par kilogramme de poids corporel total par jour (mg/kg/jour).

14. Produit selon la revendication 12 ou 13, dans laquelle le cancer est un cancer du sein, de la prostate, du colon et/ou du poumon.
